# EUROPEAN PATENT SPECIFICATION

(11) **EP 0 871 759 B1**
(45) Date of publication and mention of the grant of the patent: **14.02.2001**
(21) Application number: 96915145.5
(22) Date of filing: 15.05.1996
(51) Int. Cl.: C12P 7/10

(54) **PROCESS FOR THE PRODUCTION OF ETHANOL**
VERFAHREN ZUR HERSTELLUNG VON ETHANOL
PROCEDE DE PRODUCTION D'ETHANOL

(30) Priority: 22.05.1995 IE 950367
(43) Date of publication of application: 21.10.1998
(73) Proprietor: Maxol & C.B. Manufacturing Limited, Dublin 2 (IE); McCormack, Christopher Alphonsus, Dublin 13 (IE)
(72) Inventor: McCORMACK, Christopher, Alphonsus, Dublin 13 (IE)
(74) Representative: Casey, Lindsay Joseph
(86) International application number: IE9600031
(87) International publication number: WO9637627

(56) References cited:
- US-A- 3 990 944
- US-A- 4 220 721
- US-A- 4 224 410
- US-A- 4 409 329
- US-A- 4 490 469
- US-A- 5 100 791
- US-A- 5 258 293
- US-A- 5 372 939
- DATABASE WPI Section Ch, Week 8737 Derwent Publications Ltd., London, GB; Class C03, AN 87-261319 XP002008891 & JP 62 181 794 A (TAKARA SYUZO KK) , 10 August 1987

## Description

### Technical Field

This invention relates to the production of ethanol from vegetable material.

### Background Art

The microbial production of ethanol from fruits, grains, potatoes and the like is well known. In general, sugars are obtained from the materials either directly or, for example, by the use of a starch boiler, and the sugars are converted to ethanol by the action of yeasts such as *Saccharomyces cerevisiae.* When the sugar has been converted to ethanol, the ethanol is distilled from the reaction mixture until the required degree of purity has been obtained.

Additionally, cellulosic materials have been used to produce ethanol by chemical or enzymatic processes or by a combination of said processes. Such materials include wood, wheat straw (Biotechnology and Bioengineering (U.S.A.) (1984) Vol. 26, No. 9 page 1122-1125) and rice straws (Enzyme and Microbial Technology (England) (1985) Vol. 7, No. 3 page 115-120).

GB 2 186 289 B describes a process for producing ethanol from non-cultivated species of graminae (grasses), which process comprises the steps of: homogenising the grass; hydrolysing the homogenised grass with an inorganic base to render the cellulose in the grass susceptible to attack by β-cellulase; reacting the treated grass with β-cellulase so as to convert the cellulose in the grass to glucose; filtering the reaction medium; fermenting the filtrate with an ethanol-producing microbial system (e.g. *S.cerevisiae*); and separating the ethanol thus produced.

The process described in GB 2 186 289 B is advantageous in that it provides a commercial yield of ethanol from grasses which are widely available in countries having a wet climate and, therefore, an abundant supply of grasses.

In the prior art processes, however, the timescales involved from start to finish are relatively long. Conventional alcohol production (the process typically used by distillers) requires of the order of 3 days. The timescale of the process of GB 2 186 289 B is of the order of 30 hours (12 hours incubation with β-cellulase and 18 hours fermentation with baker's yeast).

US-A-4,409,329, US-A-4,220,721, US-A-5,372,939, US-A-5,258,293, US-A-4,490,469, US-A-4,224,410, US-A-3,990,944 and US-A-5,100,791 each disclose a process in which a vegetable material or a derivative of such a material is enzymatically treated to saccharify the material and in which the sugars so produced are converted to ethanol. Derwent WPI Accession No. 87-261319 discloses treating a biomass with an acid or base in order to provide a more suitable starting material for enzymatic conversion to glucose and ethanol.

It is an object of the present invention to reduce the timescales involved in the commercial production of ethanol from vegetable material and to increase the yield from a given mass of feedstock (or starting material).

### Disclosure of Invention

Accordingly, the invention provides a process for producing ethanol from a vegetable material, which comprises subjecting said vegetable material to an enzymatic treatment with a mixture of enzymes capable of saccharifying said vegetable material and converting the sugars so produced to ethanol, the entire process being carried out in a single reaction vessel, wherein a glucose enriched solution, obtained by the gelatinisation, dextrinisation and saccharification of a starch-rich feedstock material, is added to the reaction mixture.

By vegetable material herein is meant a material of plant origin, more particularly plants that were capable of carrying on photosynthesis.

By combining the production of sugar with the conversion of sugar to ethanol in a single reaction vessel, the number of process steps is decreased and efficiency is improved relative to prior art processes such as that described in GB 2 186 289 B.

Preferably, the mixture of enzymes will include one or more enzyme(s) selected from a cellulase, a hemicellulase or a mixture thereof.

Further, preferably, the mixture of enzymes includes one or more enzymes capable of saccharifying starch, such as one or more amylases.

The yield is also increased in relation to prior art processes because cellulose, hemicellulose and starch are all converted to ethanol. Conventionally, one will convert either the cellulose or the starch to ethanol. Certain materials low in starch but high in cellulose (e.g. grasses) would be uneconomical when used in a starch-converting process, while other materials high in starch but low in cellulose (e.g. potatoes) would be uneconomical when used in a cellulose-converting process. In the process according to the invention, either type of material can be used. Additionally, the minor proportions of cellulose or hemicellulose in a starch-rich material are not rejected or disposed of, as they are actually used to produce ethanol. The same is true of the starch in a cellulose- or a hemicellulose-rich material.

Preferably, the conversion of sugars to ethanol is carried out in the presence of an ethanol-producing microbial system.

Further, preferably, the microbial system is a yeast, more especially a yeast selected from the genera *Candida, Kluyveromyces, Pischia* and *Saccharomyces* or a mixture thereof.

Surprisingly, it has been found that the three types of enzyme hereinbefore specified are compatible in the same reaction mixture, and that the yeast is not attacked by the enzymes of the enzyme mixture (or at least is not attacked to an extent sufficient to interfere with the process).

It will be appreciated that a microorganism, such as a yeast, used in the process according to the invention replicates, whereas the enzymes do not. In order to maintain any loss of balance between the enzymes and the microbial system due to replication on the part of the latter, one can add small amounts of a proprietary antifoaming agent, such as a silicone polymer-based antifoam containing non-ionic emulsifiers of the type sold by Sigma Chemical Company Ltd. under the trade name Antifoam B emulsion, to the reaction mixture. Such antifoaming agents have not been found to affect the activity of the enzyme mixture.

Suitably, the vegetable material is subjected to a pre-treatment with an inorganic acid or base.

Preferably, the vegetable material is homogenised prior to said pre-treatment.

Further, preferably, a gas or mixture thereof is forced through the reaction mixture of vegetable material and acid/base during hydrolysis therewith. Suitably, the procedure involves aeration of the mixture.

The material is suitably homogenised by milling or pulping to an average particle size of 5 mm or less, preferably to a particle size of 1 mm or less.

This initial homogenisation has the effect of increasing the surface area of the material, allowing better reaction with the acid or base and with the reaction mixture in the reaction vessel.

Where the proportion of cellulose to hemicellulose is quite low i.e., typically cellulose to hemicellulose in a ratio of the order of 0.5:1.0, the material may be reacted with an acid. Preferably, however, where the material contains a high proportion of cellulose i.e., typically cellulose to hemicellulose in a ratio of the order of 1.5:1.0, it is reacted with a base. This is because hemicellulose can be hydrolysed either by an acid or a base, but cellulose requires hydrolysis by a base. Preferably, the base is sodium hydroxide.

Suitably, the acid or base is provided as a solution and is retained after the pre-treated material has been fed to the reaction vessel, in order to allow a hemicellulose fraction to be separated from the solution, which fraction is also added to the reaction vessel.

This confers an advantage over prior art cellulose-conversion processes. Conventionally, the hemicellulose contained in the material is not used for the production of ethanol and is generally disposed of. According to the present invention, however, both cellulose and hemicellulose are converted to ethanol, thereby increasing the yield.

As indicated above, the process further comprises the step of adding a glucose enriched solution to the reaction mixture in order to increase the yield. The glucose enriched solution is obtained by the gelatinisation, dextrinisation and saccharification of a starch-rich feedstock material. The starch-rich feedstock material can be selected from wheat grain, corn, potato tubers or rice.

The extraction of the ethanol from the reaction mixture upon completion of the process can be carried out by centrifugation, distillation or a mixture of such processes.

Suitably, the reaction mixture contains a yeast nutrient medium such as the product sold under the trade mark Tronozymol by Fermenta, Burton-on-trent, England.

Preferably, the process is carried out at a pH in the range 3.0-6.0, more especially 4.0-5.2. A particularly preferred pH has been found to be 4.6.

Preferably, the process is carried out at a temperature in the range 20-50°C, more especially in the range 25-45°C. A particularly preferred temperature has been found to be a temperature of about 35°C.

The characteristics of the enzymes and microbial systems used will determine the operating pH and other conditions. Such enzymes and microbial systems can result from genetic manipulation procedures and, accordingly, the operating conditions can vary widely.

Suitable cellulase and other cellulose degrading enzymes for use in the process according to the invention include endogluconases, for example, 1,4-[1,3;1,4]-β-D-Glucan 4-glucano-hydrolase; EC 3.2.1.4, exogluconases, for example, 1,4-β-D-glucan cellobiohydrolase; EC 3.2.1.91, or mixtures thereof, hereinafter referred to generally as cellulases.

Suitable hemicellulase and other hemicellulose degrading enzymes for use in the process according to the invention include xylose isomerase, xylanases including for example, 1,4-β-D-xylanase; EC 3.2.1.8, β-xylosidases, such as β-xylosidase; EC 3.2.1.37, α-arabinosidases, such as α-arabinosidase; EC 3.2.1.55, α-glucuronidases, such as α-glucuronidase; EC 3.2.1.31, acetylxylan esterases and ferulic acid esterases, or mixtures thereof hereinafter referred to generally as hemicellulases.

Suitable enzymes for saccharifying starch or starch degrading enzymes for use in the process according to the invention include α-amylase, β-amylase, amyloglucosidase, pullinase, or mixtures thereof.

In the case of following Example 1 the entire process took less than 3 hours to proceed from the raw material (untreated straw or grain) to the distilled ethanol product. The actual conversion of cellulose, hemicellulose and starch to ethanol occurred in approximately 1 hour. By comparison, conventional production of ethanol from grain takes of the order of 3 days, and the process described in GB 2 186 289 B takes approximately 30 hours. In effect, therefore, the present invention allows significant increases in yield over the prior art, combined with reductions in the timescale to 4-10% of the lengthy times for ethanol production in prior processes.

The ethanol produced in accordance with the invention is suitable for consumption or for use as a fuel or fuel additive.

### Brief Description of Drawing

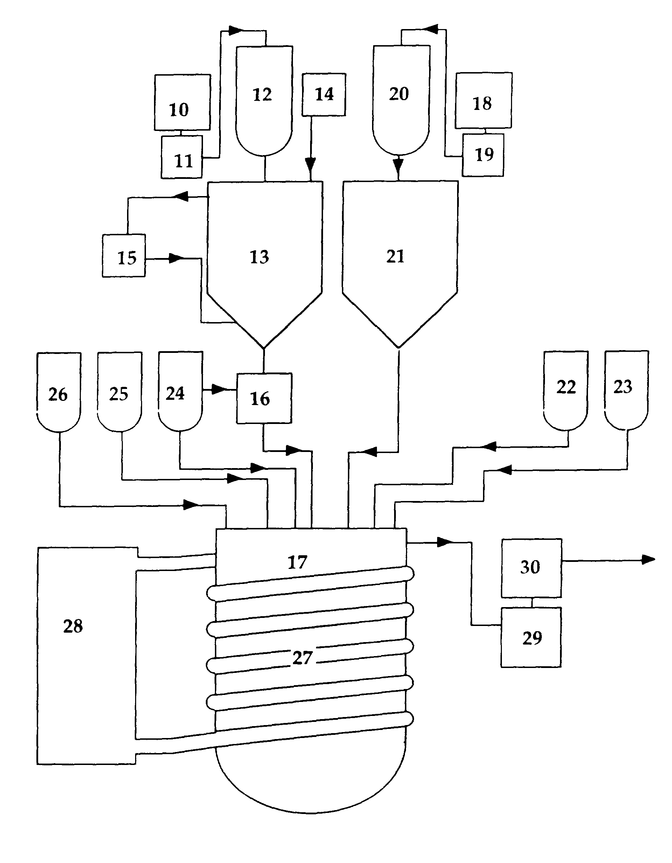

The invention will be further illustrated by the following description of an embodiment thereof given by way of example only with reference to the accompanying Figure which is a schematic representation of an apparatus for carrying out the process according to the invention.

### Modes for Carrying Out the Invention

The process according to the invention can be carried out in an apparatus in the form of a plant depicted schematically in the accompanying Figure. The apparatus comprises a bulk store 10 of the vegetable material, such as wheat straw, to be treated in accordance with the invention. The material is fed from the bulk store 10 into a mill 11 where it is mechanically milled to a suitable size such as 1 mm particle size. The milled material is dry blown using compressed air into a storage tank 12, from whence it is fed into a vertical column silo 13. The silo 13 contains a hydrolysis solution such as sodium hydroxide, which solution is replenished from a tank 14. The material is suitably mixed with the hydrolysis solution under conditions of forced air agitation supplied by a compressor 15. Following agitation, the material is allowed to settle prior to separation from the hydrolysis solution by decantation. When the hydrolysis solution is sodium hydroxide, the residual sodium hydroxide is centrifuged to recover hemicellulose-derived sugars which are retained for further processing. The treated material which has been decanted from the hydrolysis solution is fed into a holding tank 16 and the pH adjusted, as necessary. From holding tank 16, the treated mixture is pumped to a reaction vessel 17.

In the case when a glucose enriched solution is added to the reaction mixture, the feedstock material such as wheaten grain is fed from a store 18 to a mechanical crusher 19 which includes heated rollers to crack open the grains. The grain is crushed and then blown using compressed air into a storage tank 20. The crushed grain is then fed under gravity from the storage tank 20 into a standard starch boiler 21 where it is converted into a glucose enriched solution *via* gelatinisation, dextrinisation and saccharification. The glucose enriched solution is fed from the boiler 21 to the reaction vessel 17 together with the pre-treated vegetable material.

The enzyme mixture is fed from a holding tank 22 and the yeast from a holding tank 23. Oxygen is excluded from the reaction vessel 17, if it is desired that the reaction proceed anaerobically.

The reaction vessel 17 has an associated hydrochloric acid tank 24, a sodium hydroxide tank 25 and water tank 26 which are used to adjust the pH or water concentration of the reaction medium, if required.

The temperature of the reaction vessel 17 is maintained by a water-filled jacket 27 having an associated temperature control and pumping unit 28. The reaction mixture is agitated by a mechanical agitator (not shown). When the reaction has proceeded to the extent necessary, the agitation is switched off and the mixture is allowed to phase separate. The supernatant liquid (containing ethanol) is decanted off by means of a dip pump (not shown) from which it is fed to a centrifuge 29 and thence to a distillation column 30 where ethanol of the required purity is extracted.

The invention will be further illustrated by the following Examples.

### Example 1

### Preparation of ethanol from wheaten straw and grain

Ethanol was produced from wheaten straw on a laboratory scale in apparatus of the type illustrated schematically in the accompanying Figure and of the appropriate dimensions. 100 g of wheaten straw was fed from bulk store 10 into mill 11 where the straw was mechanically milled to 1 mm particle size. The milled straw was dry blown using compressed air into storage tank 12.

The milled straw was fed from storage tank 12 into vertical column silo 13. Silo 13 contained 1 litre of 0.5 M sodium hydroxide solution supplied from sodium hydroxide tank 14. The straw was added to the sodium hydroxide in an amount of 10% w/v and mixed for 1 hour with the sodium hydroxide under forced air agitation supplied by compressor 15. After 1 hour, the compressor 15 was switched off and the treated straw (containing cellulose) was allowed to settle prior to separation from the sodium hydroxide solution by decantation. The residual sodium hydroxide was centrifuged to recover the hemicellulose-derived sugars which were retained for further processing. The treated straw mixture which had been decanted from the sodium hydroxide was fed into holding tank 16 where the pH thereof was adjusted to 4.6 by the addition of hydrochloric acid, and the moisture content thereof adjusted to 30% by adding water, if necessary. This moisture content allows the straw to be pumped easily. From holding tank 16, the treated straw mixture (300-350 ml approx.) was then pumped to reaction vessel 17.

Simultaneously, 100 g of wheaten grain was fed from store 18 into mechanical crusher 19 which, as hereinbefore indicated, comprises heated rollers to crack open the grains. The grain was crushed and then blown using compressed air into storage tank 20. The crushed grain was fed under gravity from storage tank 20 into starch boiler 21 where it was converted into a sugar enriched solution (approximately 96% glucose with suspended solid particles) *via* gelatinisation, dextrinisation and saccharification. The glucose enriched solution (100-150 ml approximately) was fed from boiler 21 into reaction vessel 17. To the mixture of treated straw and glucose solution, 500-600 ml (to make 1 litre total volume) of Tronozymol (Trade Mark) solution was added. The following ingredients were then added to the reaction vessel 17:
1. 50 ml nutrient medium containing 44 g of enzymes to effect saccharification of any residual starches. The enzymes added (11 g each) were α-amylase, β-amylase, amyloglucosidase (1,4-α-D-Glucan glucohydrolase; EC 3.2.1.3) and pullulanase;
2. 100 ml nutrient medium containing cellulases and hemicellulases to effect saccharification of cellulose and hemicellulose, namely:
   a) 12 g endogluconase (1,4-[1,3;1,4]-β-D-Glucan 4-glucano-hydrolase, EC 3.2.1.4.);
   b) 8 g exoglucanase (1,4,-β-D-glucan, cellobiohydrolase, EC 3.2.1.91);
   c) 10 g cellobioase (β-D-glucoside glucohydrolase, EC 2.3.1.21); and
   d) 5 g xylanase and 5 g xylose isomerase.
3. 100 ml nutrient medium containing yeasts to convert sugars produced by the enzyme to ethanol, namely: *C.shehatae, K. marxianus, P.stipitis, P.tannophilus* and *S.cerevisae* (8 g each).

The enzymes were fed from holding tank 22 and the yeasts from a holding tank 23. Oxygen was excluded from reaction vessel 17 to allow the reaction to proceed anaerobically.

Reaction vessel 17 had an associated hydrochloric acid tank 24, sodium hydroxide tank 25 and water tank 26 which were used to adjust the pH or water concentration of the reaction medium, as required. Hydrochloric acid tank 24 was also connected to holding tank 16 and the addition of hydrochloric acid to the pre-treated straw was used to adjust the pH thereof to 4.6 prior to its addition to reaction vessel 17.

The temperature of reaction vessel 17 was maintained at 35°C by water-filled jacket 27 having an associated temperature control and pumping unit 28. Warm or cool water was pumped through jacket 27, as required, to maintain the temperature at 35°C. The reaction mixture is agitated by a mechanical agitator (not shown).

Care was taken not to introduce any oxygen. The temperature and pH were carefully controlled to ensure that both enzymes and yeasts were active under the reaction conditions. If necessary, the proprietary antifoaming agent Antifoam B emulsion (Sigma) was added to the reaction mixture if foaming occurred.

The reaction proceeded under agitation (100 r.p.m.) for approximately 1 hour, or until the ethanol concentration had reached 8% of the total volume of reaction mixture. Agitation was then switched off and the mixture was allowed to phase separate for 10 min. Phase separation and deposition of solids occurred and the supernatant liquid (containing ethanol) was decanted off by a dip pump from which it was fed to centrifuge 29 and thence to distillation column 30 where ethanol of the required purity was extracted. The residue from centrifugation and distillation can be recycled and fed back into the reaction vessel for use with the next batch. Approximately 50 g of slurry remained in the vessel after phase a separation, and this was disposed of.

Yield: 86 ml ethanol (95% purity) was produced from 100 g of straw and 100 g of grain.

It was found that the straw produced 36 ml ethanol. By comparison, the process according to GB 2 186 289 B produced 18 ml ethanol *per* 100 g grass.

The grain produced 50 ml ethanol in the present Example. By comparison conventional distillation produces approximately 30 ml alcohol *per* 100 g grain.

The process according to the invention thus provides significant increases in ethanol yield when compared to prior art processes. Furthermore, in a single process it has a higher yield than the two closest known processes combined together.

### Example 2

The process of Example 1 was repeated, with the straw being replaced by a wide range of materials in order to investigate the possible yields available from each of the materials. In each case, wheat grain was also used in order to provide a glucose enriched solution which contributes significantly to the yield of the process. The yields obtained from the materials (ignoring the amount of alcohol obtained from the grain alone) are as follows:

| Raw material | Ethanol yield (ml/100 g) |
|---|---|
| Marine algae (specify) | 61.0 |
| White office paper | 56.0 |
| Craft paper | 52.0 |
| Domestic waste paper | 51.0 |
| Milk cartons | 50.0 |
| Poster boards | 50.0 |
| Molasses (disposed of by distillers as waste) | 50.0 |
| Sugar beet | 75.0 |
| News print paper | 47.0 |
| Potatoes | 43.0 |
| Rapeseed stocks | 42.0 |
| Office waste paper | 40.0 |
| Sweet sorghum | 38.0 |
| Rice hulls | 36.0 |
| Rice straw | 31.0 |
| Hay | 29.0 |
| Silage | 11.5 |
| Bovine stomach washings (from an abattoir) | 9.7 |
| Fodder beet tops (left by animals after feeding) | 6.1 |
| Cheese whey | 4.8 |
| Sorghum straw | 2.5 |

## Claims

1. A process for producing ethanol from a vegetable material, which comprises subjecting said vegetable material to an enzymatic treatment with a mixture of enzymes capable of saccharifying said vegetable material and converting the sugars so produced to ethanol, the entire process being carried out in a single reaction vessel, wherein a glucose enriched solution, obtained by the gelatinisation, dextrinisation and saccharification of a starch-rich feedstock material, is added to the reaction mixture.

2. A process according to Claim 1, wherein the enzyme mixture includes one or more cellulase(s).

3. A process according to Claim 1 or 2, wherein the enzyme mixture includes one or more hemicellulase(s).

4. A process according to Claim 3, wherein the enzyme mixture includes one or more amylase(s).

5. A process according to any preceding claim, wherein the conversion of sugars to ethanol is carried out in the presence of an ethanol-producing microbial system.

6. A process according to Claim 5, wherein the microbial system is a yeast.

7. A process according to Claim 6, wherein the yeast is selected from the genera *Candida, Kluyveromyces, Pischia* and *Saccharomyces* or a mixture thereof.

8. A process according to any preceding claim, wherein the vegetable material is subjected to a pre-treatment with an inorganic acid or base.

9. A process according to Claim 8, wherein the vegetable material is homogenised prior to said pre-treatment.

10. A process according to any preceding claim, which is completed in a period of under 5 hours.

## Patentansprüche

1. Verfahren zur Herstellung von Äthanol aus einem pflanzlichen Material, das Folgendes umfaßt: Unterziehen von genanntem pflanzlichem Material einer enzymatischen Behandlung mit einem Enzymgemisch, das dazu in der Lage ist, genanntes pflanzliche Material zu saccharifizieren und die auf diese Weise hergestellten Zucker in Äthanol umzuwandeln, wobei das gesamte Verfahren in einem einzelnen Reaktionsgefäß durchgeführt wird, worin eine mit Glucose angereicherte Lösung, die durch die Verkleisterung, Dextrinierung und Saccharifikation eines stärkereichen Rohsubstratmaterials erhalten wird, dem Reaktionsgemisch zugefügt wird.

2. Verfahren nach Anspruch 1, worin das Enzymgemisch eine Cellulase oder mehrere Cellulasen einschließt.

3. Verfahren nach Anspruch 1 oder 2, worin das Enzymgemisch eine Hemicellulase oder mehrere Hemicellulasen einschließt.

4. Verfahren nach Anspruch 3, worin das Enzymgemisch eine Amylase oder mehrere Amylasen einschließt.

5. Verfahren nach einem der vorangehenden Ansprüche, worin die Umwandlung von Zuckern in Äthanol in Anwesenheit eines Äthanol produzierenden mikrobiellen Systems durchgeführt wird.

6. Verfahren nach Anspruch 5, worin das mikrobielle System eine Hefe ist.

7. Verfahren nach Anspruch 6, worin die Hefe aus der Gattung *Candida, Kluyveromyces, Pischia* und *Saccharomyces* oder einem Gemisch davon ausgewählt wird.

8. Verfahren nach einem der vorangehenden Ansprüche, worin das pflanzliche Material einer Vorbehandlung mit einer anorganischen Säure oder Base unterzogen wird.

9. Verfahren nach Anspruch 8, worin das pflanzliche Material vor genannter Vorbehandlung homogenisiert wird.

10. Verfahren nach einem der vorangehenden Ansprüche, welches in einem Zeitraum von unter 5 Stunden abgeschlossen wird.

## Revendications

1. Procédé de production d'éthanol avec une matière végétale, lequel comprend soumettre ladite matière végétale à un traitement enzymatique avec un mélange d'enzymes capables de saccharifier ladite matière végétale et de convertir les sucres ainsi produits en éthanol, le procédé entier étant effectué dans un seul réacteur, dans lequel une solution enrichie de glucose, obtenue par la gélatinisation, dextrinification et saccharification d'une matière de charge riche en amidon, est ajoutée au mélange de réaction.

2. Procédé selon la revendication 1, dans lequel le mélange d'enzymes inclut une ou plusieurs cellulase(s).

3. Procédé selon la revendication 1 ou 2, dans lequel le mélange d'enzymes inclut une ou plusieurs hémicellulase (s).

4. Procédé selon la revendication 3, dans lequel le mélange d'enzymes inclut une ou plusieurs amylase(s).

5. Procédé selon l'une quelconque des revendications précédentes, dans lequel la conversion des sucres en éthanol est effectuée en présence d'un système microbien producteur d'éthanol.

6. Procédé selon la revendication 5, dans lequel le système microbien est une levure.

7. Procédé selon la revendication 6, dans lequel la levure est sélectionnée des genres *Candida, Kluyveromyces, Pischia* et *Saccharomyces* ou d'un mélange de ceux-ci.

8. Procédé selon l'une quelconque des revendications précédentes, dans lequel la matière végétale est soumise à un pré-traitement avec un acide ou une base inorganique.

9. Procédé selon la revendication 8, dans lequel la matière végétale est homogénéisée préalablement audit pré-traitement.

10. Procédé selon l'une quelconque des revendications précédentes, qui est terminé en une période de moins de 5 heures.
